# EUROPEAN PATENT SPECIFICATION

(11) **EP 2 035 331 B1**
(45) Date of publication and mention of the grant of the patent: **22.05.2019**
(21) Application number: 07730001.0
(22) Date of filing: 08.06.2007
(51) Int. Cl.: C01B 25/32, A61L 27/12, A61F 2/28

(54) **NANOMETRIC HYDROXYAPATITE AND SUSPENSIONS THEREOF, ITS PREPARATION AND USE**
NANOMETRISCHER HYDROXYAPATIT UND SUSPENSIONEN DAVON, SEINE HERSTELLUNG UND VERWENDUNG
HYDROXYAPATITE NANOMÉTRIQUE ET SUSPENSIONS LA CONTENANT, SA PRÉPARATION ET SON UTILISATION

(30) Priority: 08.06.2006 IT FI20060139
(43) Date of publication of application: 18.03.2009
(73) Proprietor: Colorobbia Italia S.p.a., 50053 Sovigliana Vinci (IT)
(72) Inventor: BALDI, Giovanni, 50025 Montespertoli (IT); BITOSSI, Marco, 50056 Montelupo Fiorentino (IT)
(74) Representative: Palladino, Saverio Massimo
(86) International application number: PCT/EP2007/055642
(87) International publication number: WO 2007/141324

(56) References cited:
- EP-A2- 0 269 385
- WO-A-02/087747
- WO-A-03/000588
- WO-A-2005/110339
- FR-A1- 2 537 558
- YI ZUO ET AL: "Influence of ethylene glycol on the formation of calcium phosphate nanocrystals" JOURNAL OF MATERIALS SCIENCE & TECHNOLOGY EDITORIAL BOARD CHINA, vol. 19, no. 6, 2003, pages 628-630, XP008076402 ISSN: 1005-0302
- Y.M. Jiang ET AL: "The Effect of Different Surface Modification Agents on the Dispersion of Nano-Hydroxyapatite (n-HA) Crystallites", Key Engineering Materials, vol. 284-286, 1 January 2005 (2005-01-01), pages 55-58, XP55393039, Aedermannsdorf ISSN: 0252-1059, DOI: 10.4028/www.scientific.net/KEM.284-286.55

## Description

### Field of the invention

The present invention relates to the field of materials in nanometric form, and in particular to suspensions of said materials in liquid solvents.

### State of the art

One of the most important aims of research in the biomaterials field is the development of solid compositions usable as materials for treating bone injuries or constructing bone prostheses.

In this regard, calcium- and phosphate-based ceramic materials have aroused particular interest, in view of their bioactive and biocompatible properties.

The aforesaid materials include hydroxyapatite, octacalcium phosphate [OCP, Ca₈H₂(PO₄)₆], tricalcium phosphate [TCP, Ca₃(PO₄)₂], dicalcium phosphate dihydrate [DCPD, CaHPO₄.2H₂O] and dicalcium phosphate [DCP, Ca₂P₂O₇]. Of the aforesaid compounds, hydroxyapatite and tricalcium phosphate are the elements most often used as bone substitutes.

Stoichiometric hydroxyapatite (HAP) is represented by the chemical formula Ca₁₀(PO₄)₆(OH)₂ with a calcium/phosphorus atomic ratio of 1.67. This material is mainly used for producing artificial bones and teeth, and bone, dental and orthopaedic grafts, etc.

The paper "Influence of ethylene glycol on the formation of calcium phosphate nanocrystals", Yi Zuo et al., Journal of Materials Science and Technology, Editorial Board China, vol. 19, no. 6 (2003), pages 628-630, describes a synthesis route of calcium phosphate using calcium hydroxide Ca(OH)₂ and H₃PO₄ as reagents in ethylene glycol as solvent, at a pH value of 7.0-8.0.

The paper "The Effect of Different Surface Modification Agents on the Dispersion of Nano-Hydroxyapatite (n-HA) Crystallites", Y.M. Jiang et al., Key Engineering Materials, Vols. 284-286, pp. 55-58, 2005 describes a process in which HA nanoparticles are first produced starting from water solutions of calcium nitrate and (NH₄)₂HPO₄ with a pH adjusted between 11-12, with no organic compound used during the preparation; the thus obtained particles are then suspended in organic solvents, such as DMF or PLA, with the aid of polymeric water miscible surfactants such as PEG 6000 (MW 6000 Da).

Although the biocompatible and bioactive properties of micrometric hydroxyapatite are well known, it has the drawback of being very poorly bioreabsorbable and hence of slowing bone regeneration. Reabsorbability of HAP can be improved with ionic doping agents or by reducing the crystals to a nanometric size.

It would therefore be of great importance to be able to provide a hydroxyapatite which, once used in bone implants, is bioreabsorbable and hence replaced in time by regenerated natural bone.

### Brief description of the figures

Fig. 1 and 2 - Show respectively STEM and FEG images of the nanoparticles in suspension.
Fig. 3 - Shows a FEG image of spheroid-shaped aggregates 5µm in size.
Fig. 4 - Shows a FEG image of rounded sub-structures 1000 nm in size.
Fig. 5 - DLS analysis of the hydroxyapatite suspension obtained directly from synthesis.
Fig. 6 - DLS analysis of the hydroxyapatite powder dried by spray drying then resuspended in water.

### Detailed description of the invention

The present invention enables the aforesaid problems to be overcome by virtue of a hydroxyapatite in the form of nanometric particles which can be easily suspended in liquid solvents and then used in bone implants.

The invention also relates to a process for preparing hydroxyapatite in nanometric form and to its use in bone implantology.

In accordance with the present invention hydroxyapatite has the stoichiometric formula Ca₁₀(PO₄)₆(OH)₂, possibly doped with Mg, Si, Ti, Ce, Zn, Ag atoms In the case of doped hydroxyapatite, the aforesaid atoms are preferably present in a quantity equal to or less than 4% by weight on the total weight.

The hydroxyapatite in accordance with the invention comprises particles having dimensions between 60 and 150 nm in length and between 10 and 60 nm in width, preferably between 80 and 100 nm in length and between 20 and 40 nm in width. The aforesaid particles are preferably suspended in suitable solvents i.e. glycols such as monopropylene glycol, diethylene glycol, polyethylene glycol, polypropylene glycol etc.

The aforesaid suspensions are obtained directly during the hydroxyapatite nanoparticle preparation process.

In accordance with the invention the hydroxyapatite particles are preferably prepared starting from an aqueous solution of an appropriate precursor such as calcium acetate or another inorganic calcium salt, to which a solvent of diethylene glycol (DEG; BP = 240 °C), monopropylene glycol (PG; BP = 198 °C) or polyethylene glycol 200-600 (PEG 200-600; BP = 250 °C) type is added having a complexing ability with HAP particles.

Phosphoric acid is then added to the aforesaid clear solution followed by a basic agent so as to maintain the pH around neutrality.

Finally, the opalescent suspension obtained is heated, preferably to between 50 °C and 120 °C, for the time needed to grow the nuclei generated in the phase in which the suspension becomes opalescent, the time normally being between 8 and 24 hours.

The particles obtained in suspension have dimensions between 80 and 100 nm in length and between 20 and 40 nm in width, and remain in suspension without precipitating or flocculating. If preferred, powders can be obtained from the nanoparticulate suspension by drying by spray drying or by centrifugation, subsequent filtration under vacuum, washing with water and acetone then oven drying at 100 °C. The powders could possibly be resuspended in a suitable solvent (such as water, physiological liquid, etc); in this operation the aggregates surprisingly redisperse at the time of use into the nanometric particles of which they are composed.

The aforesaid precursors include for example water soluble Ca or Mg salts such as acetates, chlorides, nitrates, hydroxides, while preferred complexing solvents are glycols such as diethylene glycol or monopropylene glycol.

In order to maintain the pH constant at values of around 7, suitable bases can be used such as a 32% ammonia solution as well as solutions of ammonium phosphate (NH₄)₂HPO₄ 0.1 - 0.3M and NaOH 0.1-2.5 M.

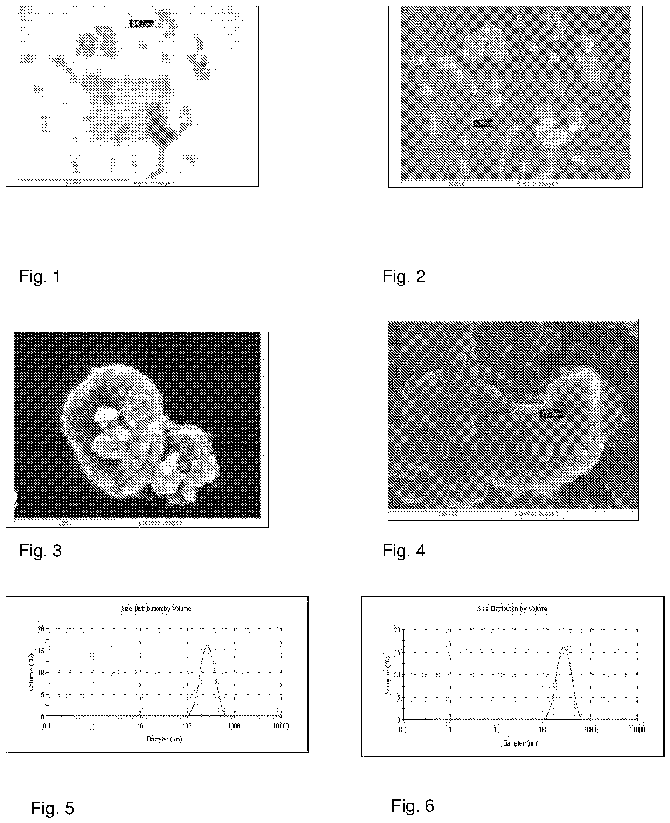

In accordance with a particular embodiment of the invention the suspensions as aforedescribed can be spray dried to enable spheroid particles of about 5 µm in diameter to be obtained together with round sub-structures of about 80-100 nm composed in turn of smaller structures 10-20 nm in size, as shown in the images (Fig 3-4).

Spray drying is conducted with an air inlet temperature of 240 °C and an outlet temperature of 160 °C. In addition to precisely regulating process parameters, this allows high boiling solvents (BP > 200 °C) to be removed and powders consisting of soft aggregates of controlled shape and size to be surprisingly obtained. The salient characteristic of these aggregates is that once suspended in suitable solvents such as water, glycols, alcohols etc, for example by high energy (e.g. ultrasound) mixing or dispersing, they give rise to the original suspension of nanoparticles (Figs. 5-6).

The hydroxyapatite thus obtained, by virtue of its distinctive morphology, is suited to sinterization processes for preparing scaffolds etc.

### Example 1

### Synthesis of hydroxyapatite (2.4% w/w concentrated suspension)

### Step 1

6 g of Ca(CH₃COO)₂•xH₂O (Riedel-de Haen, equal to 93%w/w Ca(Ac)₂) equivalent to 0.036 mol of Ca²⁺ are dissolved in 24 ml of water in a 500 ml flask equipped with a shaker, temperature probe and bulb condenser provided with a tap for distillation. 120 g of diethylene glycol (DEG) are then added. 2.46 g of a H₃PO₄ solution (Aldrich, 85% w/w solution) equal to 0.0216 mol of P are slowly added to the clear solution thus obtained. After adding H₃PO₄ a slight opalescence was observed at pH 6. The pH value was raised to 7 by slowly adding 6 ml of a 32% NH₃ solution (Merck). The system was maintained under agitation for 16 hours at a temperature of 80 °C, obtaining a yellowish-white suspension of pH 6-7.

The material obtained has a Ca/P ratio of 1.67/1 and a Ca₁₀(PO₄)₆(OH)₂ content of 2.4% w/w.

The suspension is stable even at concentrations greater than 2.4% w/w of HAP, up to 8% w/w HAP. These were obtained by distillation under vacuum.

### Step 2

### Obtaining hydroxyapatite powder

The suspension obtained in step 1 was dried using a spray dryer modified to attain high temperatures and with the following settings: Inlet 250 °C; Aspirator 100%; Pump velocity; 10%; Flow velocity 50 mm.

A white powder collects in the cyclone. This powder consists of soft aggregates of HAP nanocrystals (photo 6) easily redispersible in liquids such as water, alcohols, glycols.

## Claims

1. Process for preparing a suspension of hydroxyapatite in the form of nanometric particles **characterised in that** it comprises the following steps:
- adding diethylene-glycol, monopropylene-glycol or polyethylene-glycol of molecular weight in the range 200-600 to an aqueous solution of calcium acetate;
- adding phosphoric acid to the clear solution thus obtained then adding a basic agent in order to maintain the pH around neutrality, followed by heating the so obtained opalescent suspension to between 50 °C and 120 °C for the time needed to grow the previously generated nuclei;
- allowing the suspension to cool.

2. Process according to claim 1 wherein diethylene-glycol is used in the first step.

3. Process according to claims 1 and 2 wherein said basic agent is chosen from: 32% ammonia solution, 0.1 - 0.3 M solution of ammonium phosphate (NH₄)₂HPO₄ and 0.1 - 2.5 M NaOH.

4. Process according to claims 1 to 3, further comprising the steps of:
- drying the resulting suspension to obtain aggregates of nanometric particles of hydroxyapatite;
- resuspending the thus obtained dry aggregates of nanometric particles in a solvent selected between water and a physiological liquid, obtaining a suspension of said nanometric particles of hydroxyapatite.

5. Suspension of hydroxyapatite in the form of nanometric particles obtained from the process according to claim 1.

6. Suspension of hydroxyapatite according to claim 5 wherein said particles have dimensions between 60 and 150 nm in length and between 10 and 60 nm in width.

7. Suspension of hydroxyapatite according to claim 6 wherein said particles have dimensions between 80 and 100 nm in length and between 20 and 40 nm in width.

8. Use of a hydroxyapatite suspension according to claims 5 to 7 as material for treating bone injuries or constructing bone prostheses.

## Patentansprüche

1. Verfahren zur Herstellung einer Suspension von Hydroxyapatit in Form nanometrischer Partikel, **dadurch gekennzeichnet, dass** es die folgenden Schritte umfasst:
- Hinzufügen von Diethylenglykol, Monopropylenglykol oder Polyethylenglykol mit einem Molekulargewicht im Bereich von 200-600 zu einer wässrigen Lösung von Calciumacetat;
- Hinzufügen von Phosphorsäure zu der so erhaltenen klaren Lösung, dann Hinzufügen eines basischen Mittels, um den pH-Wert nahe der Neutralität zu halten, gefolgt von Erhitzen der so erhaltenen opaleszenten Suspension auf zwischen 50 °C und 120 °C für die Zeit, die zum Wachstum der vorher erzeugten Kerne benötigt wird;
- Abkühlenlassen der Suspension.

2. Verfahren nach Anspruch 1, wobei im ersten Schritt Diethylenglykol verwendet wird.

3. Verfahren nach den Ansprüchen 1 und 2, wobei das basische Mittel ausgewählt ist aus: 32%iger Ammoniaklösung, einer 0,1 - 0,3 M Lösung von Ammoniumphosphat (NH₄)₂HPO₄ und 0,1 - 2,5 M NaOH.

4. Verfahren nach den Ansprüchen 1 bis 3, weiterhin umfassend die Schritte:
- Trocknen der erhaltenen Suspension, um Aggregate nanometrischer Hydroxyapatit-Partikel zu erhalten;
- Resuspendieren der so erhaltenen trockenen Aggregate nanometrischer Partikel in einem Lösungsmittel, das ausgewählt ist zwischen Wasser und einer physiologischen Flüssigkeit, wobei eine Suspension der nanometrischen Hydroxyapatit-Partikel erhalten wird.

5. Suspension von Hydroxyapatit in Form nanometrischer Partikel, die durch das Verfahren nach Anspruch 1 erhalten wurden.

6. Suspension von Hydroxyapatit nach Anspruch 5, wobei die Partikel Ausdehnungen zwischen 60 und 150 nm in der Länge und zwischen 10 und 60 nm in der Breite haben.

7. Suspension von Hydroxyapatit nach Anspruch 6, wobei die Partikel Ausdehnungen zwischen 80 und 100 nm in der Länge und zwischen 20 und 40 nm in der Breite haben.

8. Verwendung einer Hydroxyapatit-Suspension nach den Ansprüchen 5 bis 7 als Material zur Behandlung von Knochenverletzungen oder zur Konstruktion von Knochenprothesen.

## Revendications

1. Procédé pour préparer une suspension d'hydroxyapatite sous la forme de particules nanométriques **caractérisé en ce qu'**il comprend les étapes suivantes :
- l'ajout de diéthylène-glycol, de monopropylène-glycol ou d'un polyéthylène-glycol de poids moléculaire situé dans la plage allant de 200 à 600 à une solution aqueuse d'acétate de calcium ;
- l'ajout d'acide phosphorique à la solution limpide ainsi obtenue puis l'ajout d'un agent basique afin de maintenir le pH aux alentours de la neutralité, suivi par le chauffage de la suspension opalescente ainsi obtenue entre 50 °C et 120 °C pendant la durée nécessaire pour la croissance des germes précédemment générés ;
- le fait de permettre à la suspension de refroidir.

2. Procédé selon la revendication 1 dans lequel du diéthylène-glycol est utilisé dans la première étape.

3. Procédé selon les revendications 1 et 2 dans lequel ledit agent basique est choisi parmi : une solution d'ammoniac à 32 %, une solution de 0,1 à 0,3 M de phosphate d'ammonium (NH₄)₂HPO₄, et 0,1 à 2,5 M de NaOH.

4. Procédé selon les revendications 1 à 3, comprenant en outre les étapes de :
- séchage de la suspension résultante pour obtenir des agrégats de particules nanométriques d'hydroxyapatite ;
- remise en suspension des agrégats secs de particules nanométriques ainsi obtenus dans un solvant sélectionné entre l'eau et un liquide physiologique, pour obtenir une suspension desdites particules nanométriques d'hydroxyapatite.

5. Suspension d'hydroxyapatite sous la forme de particules nanométriques obtenue à partir du procédé selon la revendication 1.

6. Suspension d'hydroxyapatite selon la revendication 5 dans laquelle lesdites particules ont des dimensions entre 60 et 150 nm de longueur et entre 10 et 60 nm de largeur.

7. Suspension d'hydroxyapatite selon la revendication 6 dans laquelle lesdites particules ont des dimensions entre 80 et 100 nm de longueur et entre 20 et 40 nm de largeur.

8. Utilisation d'une suspension d'hydroxyapatite selon les revendications 5 à 7 en tant que matériau pour traiter les lésions osseuses ou construire des prothèses osseuses.
